# EUROPEAN PATENT APPLICATION

(11) **EP 4 806 851 A1**
(43) Date of publication of application: **16.09.2026**
(21) Application number: 26161906.8
(22) Date of filing: 03.03.2026
(51) Int. Cl.: C02F 1/00, G01N 33/18, C02F 9/00, G01N 35/10

(54) **SAMPLING APPARATUS, SAMPLING CONTROL METHOD AND CONTROL PROGRAM FOR A WATER TREATMENT SYSTEM**

(30) Priority: 11.03.2025 JP 2025038125
(71) Applicant: Yokogawa Electric Corporation, Musashino-shi, Tokyo 180-8750 (JP)
(72) Inventor: MATSUI, Yasuhiro, Musashino-shi, Tokyo, 180-8750 (JP)
(74) Representative: Winter, Brandl - Partnerschaft mbB

(57) **Abstract**

A sampling apparatus (70) includes a first pipe (71) into which supplied water flows, a first valve (718) between an analysis apparatus (74) that performs analysis of the water, a second pipe (72) into which treated water flows, and a second valve (728) between the analysis apparatus (74) and the second pipe (72).

The apparatus enables alternating sampling of supplied and treated water without requiring two separate analysis devices.

## Description

### BACKGROUND

The present disclosure relates to a sampling apparatus, a control method, a control program, and a water treatment system.

A water treatment system (water treatment device) for producing industrial water, drinking water, and ultrapure water by performing advanced water treatment driven by autonomous optimization on recycled sewage water has been known. In recent years, autonomous operation of water treatment systems using artificial intelligence (AI) have been achieved.
Patent Literature 1: Japanese Laid-open Patent Publication No. 2020-025943
Patent Literature 2: Japanese Laid-open Patent Publication No. 2020-065964
Patent Literature 3: Japanese Laid-open Patent Publication No. 2019-010614
Patent Literature 4: Japanese Laid-open Patent Publication No. 2023-121593

The conventional technique however has a problem in that it is not possible to efficiently acquire information on quality of water in the water treatment system in some cases.

For example, in order to measure water quality of both supplied water that is supplied to filtration membranes and treated water having been filtered with the filtration membranes in the conventional water treatment system, analysis apparatuses corresponding to the supplied water and the treated water, respectively, that is, two analysis apparatuses are necessary. For this reason, with the conventional water treatment system, the cost and operations that a user bears are high and heavy and thus it is not possible to acquire information on quality of water efficiently.

An object of the disclosure is to acquire information on quality of water efficiently in a water treatment system.

### SUMMARY

According to an aspect of an embodiment, a sampling apparatus includes a first pipe into which water flows, a first valve between an analysis apparatus that performs analysis on the water that flows into and the first pipe, a second pipe into which water flows, and a second valve between the analysis apparatus and the second pipe.

According to an aspect of an embodiment, a control method performed by a computer to control a sampling apparatus including a first pipe into which water flows, a first valve between an analysis apparatus that performs analysis on the water that flows into and the first pipe, a second pipe into which water flows, and a second valve between the analysis apparatus and the second pipe, wherein the method includes controlling opening or closing the first valve and the second valve.

According to an aspect of an embodiment, a control program that causes a computer to control a sampling apparatus including a first pipe into which water flows, a first valve between an analysis apparatus that performs analysis on the water that flows into and the first pipe, a second pipe into which water flows, and
a second valve between the analysis apparatus and the second pipe, wherein the computer is caused to control opening or closing the first valve and the second valve.

According to an aspect of an embodiment, a water treatment system includes an equipment system including each water treatment device that executes a water treatment process and a control apparatus that executes control on the each water treatment device, and a management system that includes a management apparatus that determines content of control on the water treatment process using a virtual system having virtualized the each water treatment device and that controls the water treatment process of the equipment system based on the content of control via the control apparatus of the equipment system, wherein the water treatment device includes a sampling apparatus including a first pipe into which water flows, a first valve between an analysis apparatus that performs analysis on the water that flows into and the first pipe, a second pipe into which water flows, and a second valve between the analysis apparatus and the second pipe, and the control apparatus includes a processor that performs control of opening or closing the first valve and the second valve.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a diagram illustrating an overall configuration of a water treatment system according to a first embodiment;
FIG. 2 is a diagram illustrating an architecture of a water treatment system of a referential technique;
FIG. 3 is a functional block diagram illustrating a functional configuration of the water treatment system according to the first embodiment;
FIG. 4 is a front view of a sampling apparatus according to the first embodiment;
FIG. 5 is a side view of the sampling apparatus according to the first embodiment;
FIG. 6 is a diagram illustrating a configuration of the sampling apparatus according to the first embodiment;
FIG. 7 is a flowchart illustrating a flow of a process that is executed by an equipment system;
FIG. 8 is a flowchart illustrating a flow of a process that is executed by a management system;
FIG. 9 is a diagram illustrating an architecture of a water treatment system according to a second embodiment;
FIG. 10 is a diagram illustrating an architecture of a water treatment system according to a third embodiment; and
FIG. 11 is a diagram illustrating an example of a hardware configuration.

### DESCRIPTION OF THE EMBODIMENTS

A sampling apparatus, a control method, a control program, and a water treatment system disclosed by the present application will be described in detail below according to the accompanying drawings. Note that the embodiment does not limit the disclosure. The same elements are denoted with the same reference numerals and redundant description will be omitted as appropriate. It is possible to combine each embodiment as long as no inconsistency is caused.

### About Water Treatment System

A water treatment system used in a water purification plant and the like is configured by a combination of systems (level 0) having four types of functions that directly have an effect on water not to be treated. Note that the devices that execute the four types of functions include: a device (membrane) that executes treatment of physically filtering water; a device (UV/Cl/Ozone) that executes treatment of chemically oxidizing or disinfecting water; devices (mechanical components) that control conveyance of water, such as a pipe, a pump, and a valve; and a device (control H&S) including a measurement device and software necessary to control quality of water, an amount of water, a water temperature, a water pressure, etc.

In such a water treatment system, safe operations are performed by executing various instructions from a programmable logic controller (PLC) located at a higher level than each of the above-described devices to each of the devices located at a lower level according to a logic in each unit of control using the PLC.

In recent water treatment systems, operation control using artificial intelligence (AI) has been achieved. For example, in the operation control using AI, terminal devices that are systems at Level 0 are put together in stages gradually to a system at a higher level constitutively and also as input/output data and, lastly, an AI engine (machine learning model) simulates human thought and derives an optimized solution. According to the solution, an instruction is transmitted to each device.

The above-described water treatment system, however, is a system configured at Level 1, Level 2, or lower and is controlled by sequence control by the PLC and PID (P: proportion, I: integration, and D: differentiation) control is commonly used as the sequence control. In this case, while it is possible to assign an operation value corresponding to a load that the system encounters and execute a command to stop the operation and to avoid a trouble, automated operation using AI is not taken into consideration for the design and thus it it hard to say that optimal automated operation has been achieved.

In contrast to this, according to the water treatment system of the embodiment, it is possible to achieve optimal automated operation.

### First Embodiment

### Overall Configuration (Architecture)

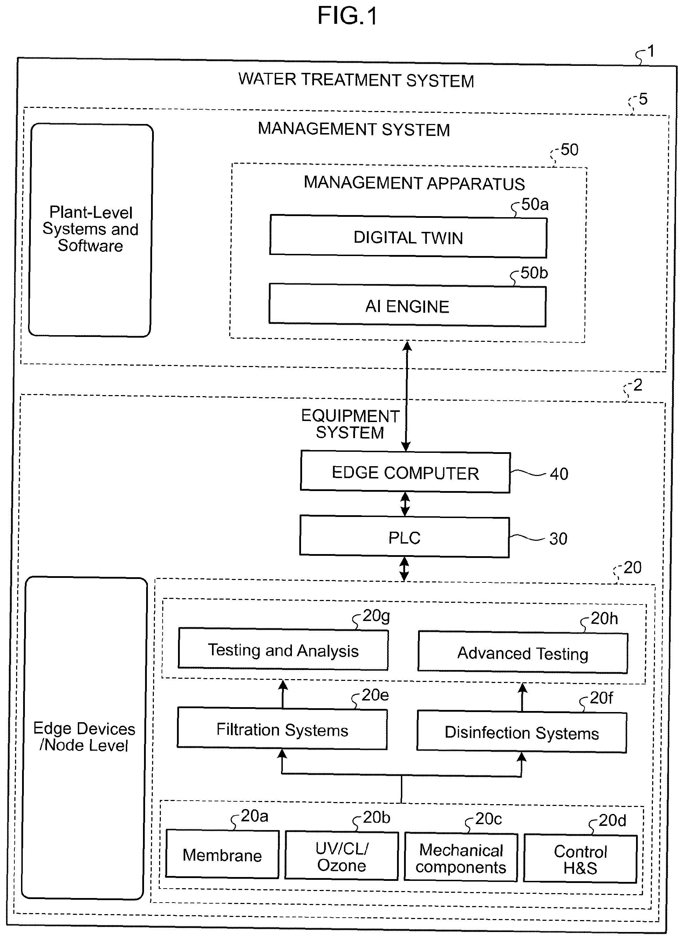

FIG. 1 is a diagram illustrating an overall configuration of a water treatment system according to a first embodiment. A water treatment system 1 illustrated in FIG. 1 is an example of a system that generates drinking water from wastewater, such as sewage and rainwater, by executing advanced water treatment. The water treatment system 1 is roughly classified into two categories. One is a management system 5 corresponding to plant-level-system and software and the other is an equipment system 2 corresponding to edge device/node level constituting actual equipment (actual system).

The equipment system 2 includes each water treatment device that executes a water treatment process and is connected to the management system 5 via a network N. Note that, various networks, such as a dedicated line, a local area network (LAN), a virtual local area network (VLAN), and the Internet can be employed as the network N.

The equipment system 2 includes a water treatment device group 20, a PLC 30, and an edge computer 40. The water treatment device group 20 includes edge devices at a node level. Here, the edge devices at a node level include membranes 20a, a UV/Cl/Ozone 20b, mechanical components 20c, control H&S 20d, filtration systems 20e, disinfection systems 20f, testing and analysis 20g, and an advanced testing 20h. Note that these devices are modularized and each of the devices is controllable.

The membranes 20a are devices that execute treatment of physically filtering water. The UV/Cl/Ozone 20b is a device that executes treatment of performing chemical accelerated oxidation or disinfection on water. The mechanical components 20c are devices that control conveyance of water, such as pipes, pumps, and valves. The control H&S 20d is a device including a measurement device and software necessary to control quality of water, an amount of water, a water pressure, a water temperature, etc.

The filtration systems 20e is a device (system) that is configured by any combination of the above-described devices 20a to 20d and that executes filtration on water. The disinfection systems 20f are devices (system) that are configured by any combination of the devices 20a to 20d and that execute disinfection on water using ultraviolet rays, heat, and the like. The testing and analysis 20g is a device that executes general testing and analysis of pH, electric conductivity, turbidity, water temperature, ultraviolet absorbance, chemical oxygen demand (COD), nitrogen (ammonia nitrogen, nitrate nitrogen, and total nitrogen), and the like, whereas the filtration systems 20e and the disinfection system 20f are devices (systems) that perform physical or chemical water treatment or a water treatment process thereof. The testing and analysis 20g has functions of measurement related to mechanical process management (e.g., on/off of pump and open/close of valve) and operation quality management (e.g., amount of water, water pressure, and quality of water) and transmission and transfer of an analog or digital signal. The advanced testing 20h is a device that executes advanced testing and analysis, such as polymerase chain reaction (PCR), total organic carbon (TOC), and adenosine tri-phosphate (ATP), on water that has been subjected to any one of or both of filtration and disinfection by any one of or both of the filtration system 20e and the disinfection systems 20f. The advanced testing 20h has functions of measurement related to management of quality of water that is safe to human body in operation quality management and transmission and transfer of an analog or digital signal.

The PLC 30 is an example of a computer that performs the above-described sequence control on each of the devices at Level 0, Level 1, Level 2, or lower. The PLC 30 executes various instructions to each water treatment device accordance to an instruction from the edge computer 40 located at a higher level.

The edge computer 40 executes operation control on the water treatment process in the equipment system 2. For example, the edge computer 40 receives data on a control status, content of control, a result of control, a treatment status, and the like, of the water treatment process from the PLC 30, executes simulation using the data and prediction using a machine learning model using the data, and acquires a result of predicting a state of the water treatment process, and the like. The edge computer 40 then notifies the PLC 30 of the content of control in order to, for example, improve the water treatment process, improve the quality, reduce costs, and adjust a volume of production by using the result of predicting a state of the water treatment process.

The management system 5 is an example of a computer that controls the water treatment process by controlling the entire equipment system 2 and includes a plant-level system and software. The management system 5 includes a management apparatus 50. The management system 5 includes a digital twin 50a that generates a virtual module (virtual system) simulating a system configured at a level of actual equipment and an AI engine 50b that causes the digital twin 50a to operate and that controls the equipment system 2, which is the actual equipment. Note that the management system 5 can be implemented by a physical machine including a memory and a processor, cloud computing, and the like. The management apparatus 50 can also be implemented by a physical machine, or can be implemented by a virtual machine, a container, and the like, using virtual technology.

The digital twin 50a collects various types of information from the equipment system 2 and each apparatus in physical space and, using the various types of collected information, reproduces the physical space in virtual space. In other words, the digital twin 50a virtually configures a virtual system that simulates the same variation as that of the equipment system 2, which is actual equipment. The virtual system simulated by the digital twin 50a includes elements of each water treatment device and a virtual terminal device or a virtual module obtained by combining the elements. The virtual module includes a virtual water treatment device corresponding to each water treatment device of the equipment system 2, which is actual equipment.

In the virtual system, it is possible to set and update a parameter enabling direct or indirect calculation of the causal relation between input and output to and from the virtual terminal device or the virtual module based on preliminarily collected data on actual equipment and data sequentially transmitted from the actual equipment. The AI engine 50b to be described below sets and updates the parameter.

Note that the digital twin 50a is also able to form a virtual system by freely combining virtual water treatment devices of a module or virtual modules.

Regarding the virtual system on the digital twin 50a, the AI engine 50b is able to derive a virtual operation condition optimal for various environments and calculate an input/output value by collating the virtual module with not only a configuration simulating the actual equipment but also a module including individual virtual water treatment devices. The AI engine 50b outputs the optimal virtual operation condition and the input/output value to an administrator and the like or notifies the edge computer 40 of the condition and the value to execute a parameter update.

Furthermore, on the virtual system implemented by the digital twin 50a, the AI engine 50b performs simulation on the virtual system for sudden variation and specifies an optimal system configuration. The AI engine 50b then outputs a change to the optimal system configuration to the administrator and the like and updates the parameter that the edge computer 40 uses for simulation and the like.

Furthermore, the AI engine 50b can also use, for example, a machine learning model using deep learning and the like. Note that the management apparatus 50 may be implemented by a cloud system or may be disposed in a module. The management apparatus 50 may have a function of displaying a schematic diagram representing a configuration of a virtual system, a schematic diagram representing a configuration of an actual system, and input/output values and parameters of each water treatment device, each module, each virtual water treatment device, and each water treatment module.

### Description of Reference Technique and Problems

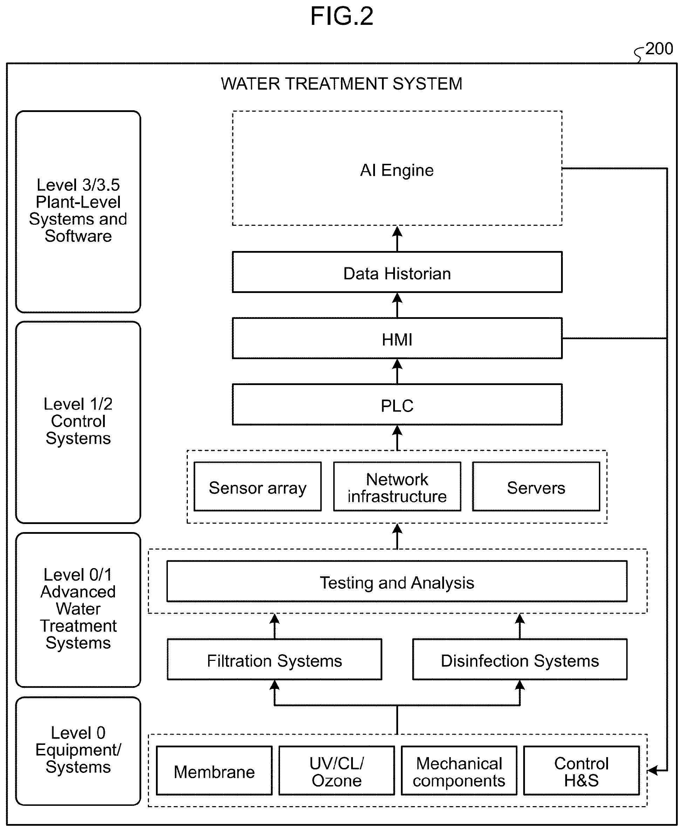

Here, a commonly used water treatment system will be described as reference technique. FIG. 2 is a diagram illustrating the architecture of a water treatment system 200 according to the reference technique. As illustrated in Figure 2, the water treatment system 200, such as a water purification plant, includes apparatuses and systems at Level 0, advanced water treatment purification systems at Level 0 and Level 1, control systems at Level 1 and Level 2, and plant-level systems and software at Level 3 and Level 3.5.

In order to control the entire system in accordance with water to be treated, an installation environment, and a purpose of treatment, the water treatment system 200 described above is constructed in a configuration in which the systems at Level 0 are combined and modularized as systems at Level 1 and Level 2 such as the filtration systems and the disinfection systems.

Furthermore, in the water treatment system 200, testing and analysis are performed on each of the modularized systems at Level 0 or systems at Level 0 and Level 1. The systems at Level 1 and Level 2 that are systems at a higher level include a sensor array, network infrastructure, and a server that controls transmission and reception of data and thereby receive data on the testing and analysis executed by the modularized systems at Level 0 or the systems at Level 0 and Level 1.

Furthermore, the systems at Level 1 and Level 2 include the PLC with functions at Level 1 and Level 2 that are integrated and a human machine interface (HMI) for putting together pieces of information collected and transmitted from the PLC and manually operating the entire systems.

In recent years, operation of further stabilized water treatment systems has been achieved by further evolving human control via the HMI and introducing a system with an artificial intelligence (AI) engine using machine learning (ML) mounted thereon as the systems at Level 3 and Level 3.5 that are much higher levels. In this case, data historian is provided in interfaces between the systems at Level 1 and Level 2 and the systems at Level 3 and Level 3.5. Control on the entire water treatment system by AI instead of a human has been achieved by causing AI to learn control that has relied on human experiences via the data historian.

As described above, terminal devices (system at Level 0) of the water treatment system 200 serving as the reference technique are put together in stages gradually to a system at a higher level constitutively and also as input/output data. In the water treatment system 200, eventually, an AI engine simulates human thought and derives an optimized solution and, using the solution, sends a command to each device (system at Level 0).

The water treatment system 200 described above, however, includes systems at Level 1, Level 2, and lower. These systems are not designed based on an idea of automated operation using AI. Before development of AI by ML, hierarchical systems obtained by putting together complicated systems in stages have been used such that a computer or a human can manage the systems. That is, a signal and behavior of a Level-0 terminal device are received to enable a grasp and management of a state by a human or data analysis, items to be managed are narrowed down, and control measures using feedback and the like have been proposed. In order to perform optimization by AI using ML in the entire water treatment system, a control loop for each hierarchy has to be optimized, and each device is not comprehensively controlled and overall optimization has been not achieved.

Furthermore, due to the hierarchization, it is far from optimization of the entire water treatment system in consideration of a combination of low-level systems, that is, Level-0 devices and expansion and replacement across hierarchies of devices. Furthermore, also from the viewpoint of cooperation of a plurality of water treatment systems, data used for AI to learn and analyze operation records in other facilities is not sufficiently accumulated because data diversion between devices is difficult. Apparatuses using a simulator have been proposed as operation support apparatuses in water treatment systems (facilities) and these apparatuses are merely simulators simulating actual equipment and thus it is hard to say that the apparatuses are ones obtained by optimizing the actual equipment as a whole.

In view of the problems of the water treatment system 200 that is used recently, in the first embodiment, a water treatment system 1 that enables optimal automated operation of the water treatment system by controlling a water treatment process from the outside of actual equipment for the edge computer 40 by using machine learning, simulation, virtualizing technology, and the like will be described.

### Functional Configuration of Water Treatment System 1

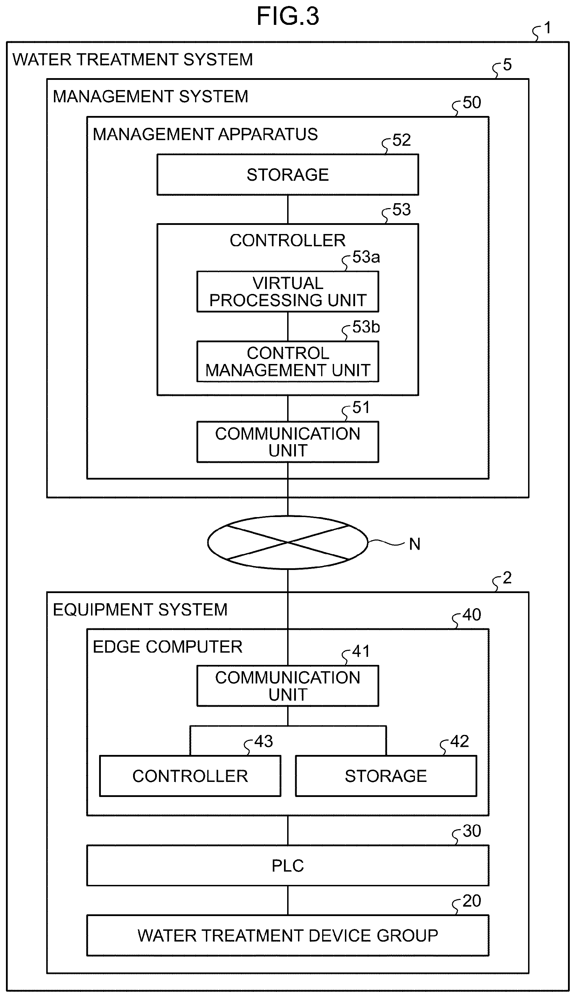

FIG. 3 is a functional block diagram illustrating a functional configuration of the water treatment system 1 according to the first embodiment. As illustrated in FIG. 3, the water treatment system 1 includes the equipment system 2 and the management system 5.

### Configuration of Equipment System 2

The equipment system 2 is a system that executes a water treatment process and includes the water treatment device group 20, the PLC 30, and the edge computer 40. The equipment system 2 does not have a hierarchized functional system configuration but constitutes a module that performs minimum operation control including individual water treatment devices and the edge computer 40. This module can also be arranged and combined so as to be directly controlled by the management system 5 (plant-level-system and software).

The water treatment device group 20 includes water treatment devices that execute water treatment processes including filtration, disinfection, piping and measurement. For example, the water treatment device group 20 includes membranes UF-RO 20a, UVAOP Ozone 20b, the mechanical components 20c, and the control H&S 20d. Note that the individual water treatment devices can be freely arranged in a module by, for example, combining the devices in parallel or in series, or arranging a plurality of devices. Moreover, the module may include not only the water treatment device but also an analysis device capable of performing sampling and analyzing quality of water and performance.

The PLC 30 is an example of an apparatus that executes various instructions and sequence control to each device in the water treatment device group 20. For example, the PLC 30 executes various types of control related to the water treatment process, such as changing water temperature, open-close control on a valve, and controlling the amount of water, on each water treatment device in accordance with a logic including control (control logic) such as PID predetermined in an initial stage such as a design stage and an operation start stage of the water treatment system 1 and then the PLC 30 transmits the result (device information) to the edge computer 40.

Furthermore, the PLC 30 executes modification, addition, change, and deletion of a logic and the like in accordance with an instruction from the edge computer 40 located at a higher level. The PLC 30 then executes various types of control, such as changing the water temperature, open-close control on a valve, and control on the amount of water, in accordance with the updated logic and the like.

The edge computer 40 is an example of an apparatus that executes operation control on the water treatment process in the equipment system 2 based on a result of operation of each water treatment device executed in units of control on the water treatment process. The edge computer 40 includes a communication unit 41, a storage 42, and a controller 43.

The communication unit 41 is a processing unit that controls communication with other apparatuses and is implemented by, for example, a communication interface and the like. For example, the communication unit 41 receives data on a water treatment process such as a state and a control result from each water treatment device in the water treatment device group 20 and receives various types of data from the management apparatus 50. The communication unit 41 transmits various types of data including a control instruction to the PLC 30 and transmits various types of data on the water treatment process and various types of data on the control of the PLC 30 to the management apparatus 50.

The storage 42 is an example of a processing unit that stores various types of data and programs executed by the controller 43, and the like, and is implemented by, for example, a memory, a hard disk, and the like.

The controller 43 is a processing unit that controls the entire edge computer 40 and is implemented by, for example, a processor. Specifically, using device information on the operation status of each water treatment device in the equipment system 2 to which the controller 43 belongs, the controller 43 executes optimization of the water treatment process in the equipment system 2 to which the controller 43 belongs.

Examples of the device information include information indicating whether or not each water treatment device is normally operating and information including the content of treatment based on current set values (e.g., amount of water, temperature, and open/close level of valve) for each water treatment device and the content of control about which an instruction has been given from the PLC 30 for each water treatment device.

Then, the controller 43 generates a first condition related to the water treatment process in the equipment system 2 based on each piece of device information. Thereafter, the controller 43 outputs processing execution, processing change, and the like to the PLC 30 in accordance with the first condition. Note that examples of the first condition include an operation condition that can be controlled in the equipment system 2, such as quality of water, time of disinfection, and a volume of production, within a range of a specification according to a condition of a client in the design stage of the equipment system 2, and the like.

In a specific example, the controller 43 acquires the status of the water treatment process, the operation status of each water treatment device, and the like from each water treatment device. The controller 43 acquires, from the PLC 30, a result of execution of a logic and the like performed by the PLC 30. Then, using each piece of acquired data, the controller 43 executes simulation or prediction using a machine learning model and acquires a prediction result of a preliminarily specified item such as a state, a risk level, and a cost of the water treatment process. Thereafter, the controller 43 executes changing the content of control and the like for the PLC 30 in accordance with the prediction result.

More specifically, the controller 43 increases the opening degree of a valve in order to increase a volume of production at the time when the volume of production is predicted to decrease or activates a temperature adjustment device in which alarm output of abnormally high temperature is predicted after several hours.

In other words, the controller 43 (edge computer 40) is able to execute control for the optimized operation of the water treatment system 1 in order to maintain the volume of production, reduce costs, and perform stable operation within a range of a specification predetermined at the time of designing the inside of the equipment system 2.

The controller 43 is also able to change or add a logic and the like of the PLC 30 by executing optimization of the water treatment process in the equipment system 2, to which the controller 43 belongs, in accordance with an instruction from the management system 5 (plant-level-system and software). For example, the controller 43 adds a logic and the like that increase an amount of water for increasing the volume of production, changes a threshold of an abnormality in temperature, which has been set in an existing logic and the like, to a new threshold newly set on the side of the management system 5, and reduces a part of the logic and the like for reducing costs.

In other words, the controller 43 (edge computer 40) can also execute control for optimized operation of the water treatment system 1 beyond the range of the specification predetermined at the time of designing the inside of the equipment system 2 in accordance with an instruction of the management system 5 (plant-level-system and software). This is, however, merely one example, and the instruction from the management system 5 (plant-level-system and software) may be within the range of the specification predetermined at the time of designing the inside of the equipment system 2.

### Configuration of Management System 5

As illustrated in FIG. 3, the management system 5 includes the management apparatus 50 implemented by a physical machine or a virtual machine. The management apparatus 50 includes a communication unit 51, a storage 52, and a controller 53.

The communication unit 51 is a processing unit that controls communication with other apparatuses and is implemented by, for example, a communication interface and the like. For example, the communication unit 51 receives, from the edge computer 40, content of control by the PLC 30 and various types of data generated in the equipment system 2. The communication unit 51 transmits various types of data generated by the controller 53 to the edge computer 40.

The storage 52 is an example of a processing unit that stores various types of data, programs executed by the controller 53, and the like, and is implemented by, for example, a memory and a processor.

The controller 53 is a processing unit that controls the entire management apparatus 50 and is implemented by, for example, the processor. Specifically, the controller 53 includes a virtual processing unit 53a and a control management unit 53b and executes operation control on the water treatment process in the equipment system 2 via the edge computer 40.

The virtual processing unit 53a is a processing unit that acquires data on the operation status of the water treatment process in the equipment system 2 from the edge computer 40 and that, using each water treatment device virtualized by using the virtualizing technology and the acquired data, executes simulation of the water treatment process. That is, the virtual processing unit 53a corresponds to the digital twin 50a in FIG. 1 and virtually configures a virtual system that simulates the same variation as that of the equipment system 2, which is actual equipment.

The control management unit 53b is a processing unit that executes control on a preprocessing process in the equipment system 2 via the edge computer 40. Specifically, the control management unit 53b corresponds to the AI engine 50b in FIG. 1 and, when, for example, a change in the specification of the equipment system 2, a change in the request, or a change in the demand of the total amount of generated water occurs, the control management unit 53b executes simulation using the virtual processing unit 53a. Then, based on the result of the simulation, the control management unit 53b executes a change in a logic currently executed in the PLC 30 and the like.

For example, the control management unit 53b acquires process information on the operation status of the water treatment process from the edge computer 40 of the equipment system 2 and generates content of control that optimizes the entire equipment system 2 based on the acquired process information. The control management unit 53b then outputs the content of control to the edge computer 40 of the equipment system 2. Note that examples of the process information include the content of control about which the edge computer 40 has given an instruction to the PLC 30, the execution status of the water treatment process executed according to the content of control, and the content of simulation executed by the virtual processing unit 53a (digital twin 50a).

In an example of the optimization, using a trained machine learning model and the like, the control management unit 53b is able to execute various types of prediction related to the water treatment process in the equipment system 2. For example, using various types of data acquired, generated, and simulated by the virtual processing unit 53a (digital twin 50a), the control management unit 53b generates a second condition related to the water treatment process. Then, the control management unit 53b notifies the edge computer 40 of the content of control in accordance with the second condition. As a result, the edge computer 40 executes a change and the like in the content of control via the PLC 30.

Note that the second condition includes the content of operation and the like in accordance with information, which has been, for example, specified from the outside of the equipment system 2. For example, the control management unit 53b executes simulation by using the virtual processing unit 53a (digital twin 50a) when a change in the specification of a client occurs or improvement including costs and the like is proposed to the client outside the range of the specification of the equipment system 2. Thereafter, when the simulation result is permitted by the client or the like, the control management unit 53b outputs an instruction to change a logic and the like, the logic after the change, and the like to the edge computer 40 in accordance with the result of the simulation.

### Configuration of Sampling Apparatus

The control H&S 20d measures water quality of supply water that is supplied to the filtration systems 20e and the disinfection systems 20f or treated water that is treated by the filtration systems 20e and the disinfection systems 20f.

In order to measure the water quality of both of the supplied water and the treated water, having the control H&S 20d to include two measurement apparatuses corresponding to the supplied water and the treated water, respectively, can be considered. Meanwhile, measurement apparatuses for measuring, for example, turbidity, ultraviolet absorbance, TOC, ATP, and the like are expensive and thus preparing a plurality of measurement apparatuses increases the costs significantly.

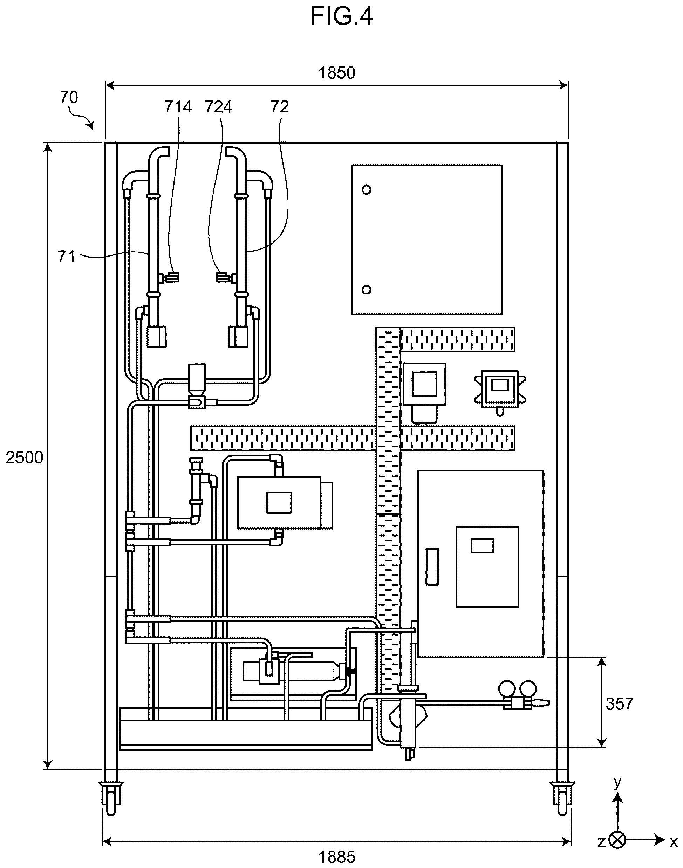

On the contrary, according to a sampling apparatus 70 illustrated in FIG. 4, it is possible to measure the water quality of both of supplied water and treated water without preparing a plurality of measurement apparatuses. FIG. 4 is a front view of the sampling apparatus according to the first embodiment.

The sampling apparatus 70 can be implemented as part of the control H&S 20d. As illustrated in FIG. 4, the sampling apparatus 70 includes a first pipe 71, a second pipe 72, a valve 714, and a valve 724.

Note that a computer enables open-close control on the valves of the sampling apparatus 70. Here, the edge computer 40 enables open-close control on the valves. The edge computer 40 transmits a signal corresponding to open or close or a signal representing an aperture to each of the valves. The valve drives according to the received signal and opens or closes. The supplied water flows into the first pipe 71. The treated water flows into the second pipe 72.

The first pipe 71 and the second pipe 72 have similar shapes. For example, in the first pipe 71 and the second pipe 72, an upright container (trunk) has a diameter of 13 mm or more. The sampling apparatus 70 has an overall length of 20 cm or more. The first pipe 71 receives inflow of the supplied water in the water treatment process. The second pipe 72 receives inflow of the treated water in the water treatment process.

FIG. 4 illustrates an example of dimensions of the sampling apparatus 70. Directions are represented by a x-axis, a y-axis and a z-axis that are orthogonal to one another. For example, the sampling apparatus 70 has a length (vertical length) of 2500 mm in the y-axis direction. For example, the sampling apparatus 70 has a length (width) of 1850 to 1885 mm in the x-axis direction.

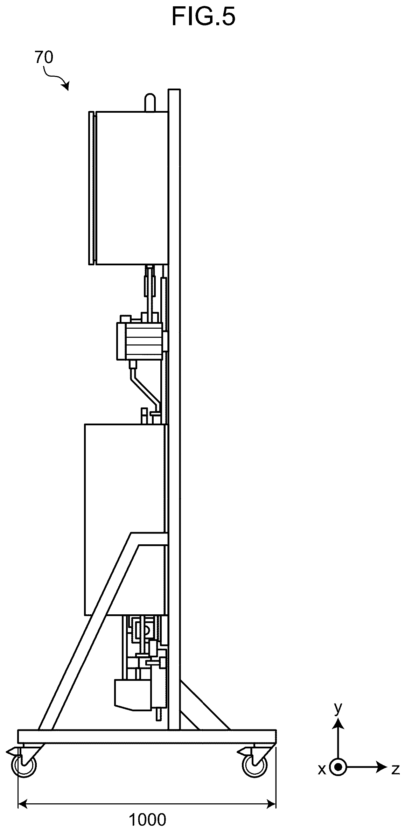

FIG. 5 is a side view of the sampling apparatus according to the first embodiment. For example, as illustrated in FIG. 5, the sampling apparatus 70 has a length (depth) of 1000 mm in the z-axis direction.

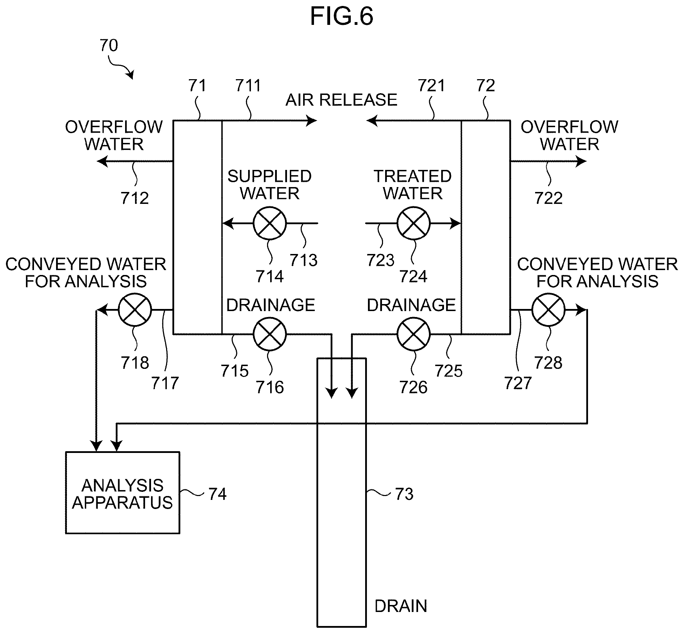

Using FIG. 6, a configuration of the sampling apparatus 70 will be described in detail. FIG. 6 is a diagram illustrating the configuration of the sampling apparatus according to the first embodiment.

As illustrated in FIG. 6, the first pipe 71 includes a pipe 711 for releasing the air, a pipe 712 through which overflow water flows, a pipe 713 through which supplied water flows, a pipe 715 for drainage, and a pipe 717 through which conveyed water for analysis flows. The valve 714 controls the water flow through the pipe 713. A valve 716 controls the water flow through the pipe 715. A valve 718 is between an analysis apparatus 74 that performs analysis on the water that flows into and the first pipe 71. The valve 718 (first valve) controls the waterflow through the pipe 717. The pipe 717 is connected to the analysis apparatus 74. The pipe 715 is connected to a pipe 73 for waste water. The analysis apparatus 74 may be the testing and analysis 20g.

As illustrated in FIG. 6, the second pipe 72 includes a pipe 721 for releasing air, a pipe 722 through which overflow water flows, a pipe 723 through which treated water flows, a pipe 725 for drainage, and a pipe 727 through which conveyed water for analysis flows. The valve 724 controls the water flow through the pipe 723. A valve 726 controls the water flow through the pipe 725. A valve 728 (second valve) is between the analysis apparatus 74 and the second pipe 72. The valve 728 controls the water flow through the pipe 727. The pipe 727 is connected to the analysis apparatus 74. The pipe 725 is connected to the pipe 73 for waste water.

The supplied water and the treated water flow into the trunk (the first pipe and the second pipe) in flow equal to or larger than an amount of drainage and in which the overflow water flows through. In order to keep detaining real-time water quality, letting the overflow water to flow out is kept promoted. Thus, while receiving a signal from a flow cell (sensor that makes it possible to check a water flow status) that is attached to an overflow pipe (the pipe 712 and the pipe 713), the edge computer 40 is able to appropriately adjust the amount the supplied water and the treated water that are conveyed water. Accordingly, the edge computer 40 performs control such that overflow water is kept caused from the first pipe 71 when the valve 718 is opened.

In order to prevent accumulation of sediment, drainage from the pipe 715 and the pipe 725 is performed. On the other hand, in view of regular internal cleaning of the trunk, degrees of opening of the valve 716 for drainage and the valve 726 are also manually adjustable.

The pipe 711 and the pipe 721 release the air in normal times and overflow the supplied water and the treated water in an emergency.

The edge computer 40 controls open/close of the valve 718 and the valve 728 according to an operational status of a system to be controlled. For example, in the case where the supplied water and the treated water in the UF membranes are to be analyzed and flow into the sampling apparatus 70, the edge computer 40 alternately performs control for closing the valve 718 and opening the valve 728 and control for opening the valve 718 and closing the valve 728 at regular intervals. This is to grasp filtration performance of the UF membranes. For example, the edge computer 40 causes the valve 718 and the valve 728 to open or close alternately in every one minute to 20 minutes by timer control.

In the case where the UF membranes are in backwashing or chemical washing, the edge computer 40 closes both of the valve 718 and the valve 728 such that analysis is not performed.

In the first pipe 71 and the second pipe 72, an ultrasonic generator may be set as a cleaner. In other words, the cleaner cleans the first pipe 71 and the second pipe 72 by ultrasound. When sampling (a flow-in of the supplied water and the treated water) is not performed, the ultrasonic generator prevents attachment of and contamination by biofilms and sediment in the trunk.

The cleaner cleans both of the first pipe 71 and the second pipe 72 with both of the valve 718 and the valve 728 being closed. When chemical cleaning on the UF membranes is being performed, the edge computer 40 may open the valve 714 and the valve 724 and close the valve 716 and the valve 726 and, while causing overflow water to flow out of the pipe 711, the pipe 712, the pipe 721, and the pipe 722, perform cleaning using the cleaner.

Furthermore, the edge computer 40 may input a signal of an open-close status of the valve 718 and the valve 728 together with a result of analysis by the analysis apparatus 74 to a machine learning model and further perform analysis. This enables the edge computer 40 to analyze a trend of water quality and the like in the case where the supplied water is to be analyzed and the case where the treated water is to be analyzed separately.

### Flow of Process

An example of a flow of a process that the equipment system 2 executes and an example of a flow of a process that the management system 5 executes will be described next.

### Process performed by Equipment System 2

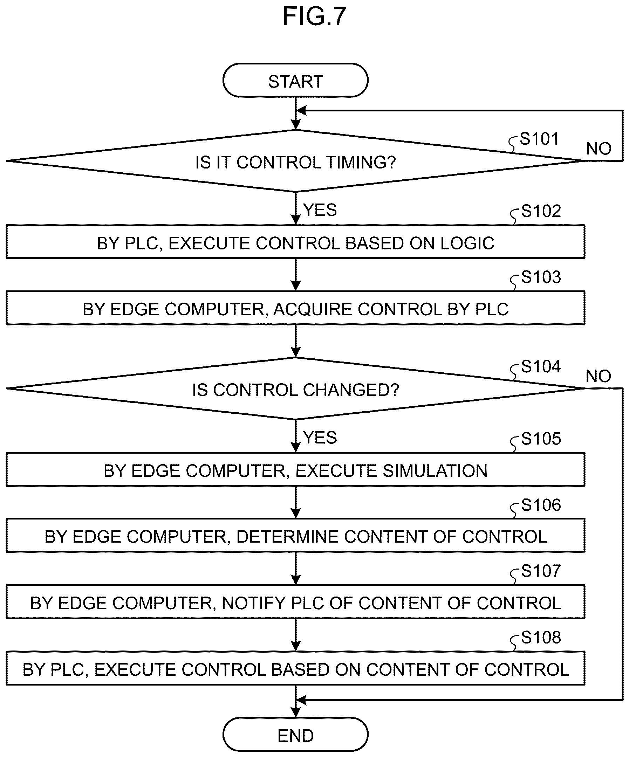

FIG. 7 is a flowchart illustrating the flow of the process executed by the equipment system 2. As illustrated in FIG. 7, when predetermined control timing is reached (S101: Yes), the PLC 30 executes control on the water treatment process based on a logic that has been set and the like (S102). The edge computer 40 then acquires the result of the control by the PLC 30 (S103), detects an abnormality, occurrence of deviation from a normal value, or the like, based on the result of the control by the PLC 30, and determines whether to change the control (S104).

On determining that the control change is necessary (S104: Yes), the edge computer 40 executes simulation and the like (S105) and determines content of control to be executed as a countermeasure (S106).

The edge computer 40 then notifies the PLC 30 of the control content (S107) and the PLC 30 makes a change and the like of the set logic and the like based on the control content of which the PLC 30 is notified and executes control on the water treatment process (S108).

### Process performed by Management System 5

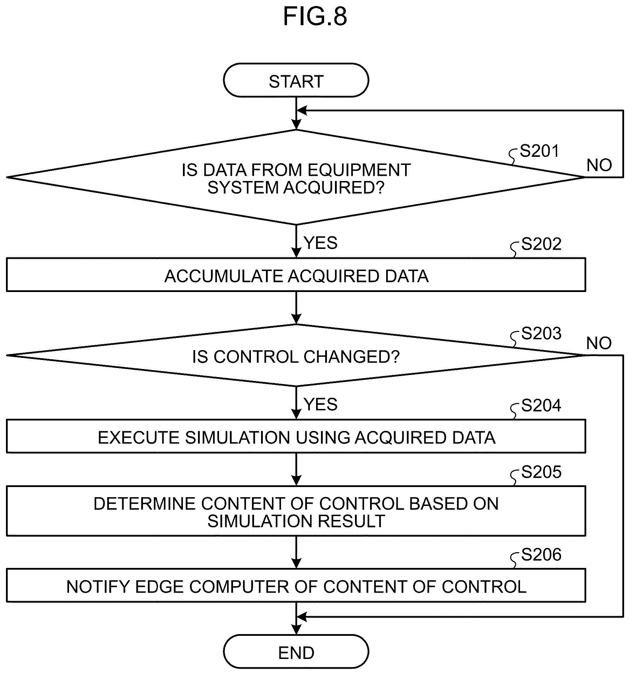

FIG. 8 is a flowchart illustrating the flow of the process executed by the management system 5. As illustrated in FIG. 8, on acquiring data of a state and a process result related to the water treatment process from the equipment system 2 via the edge computer 40 (S201: Yes), the management system 5 accumulates the acquired data (S202).

Then, when a control change including a change in the demand, a change in the setting, a change in the request, and an abnormal state occurs (S203: Yes), the management system 5 executes simulation using the acquired data and using the virtual system of the water treatment process that is generated by the virtual processing unit 53a (digital twin 50a) (S204).

Thereafter, using a table in which the simulation result is associated with a design change and the like, the management system 5 determines content of control for improving the water treatment process performed by the equipment system 2 and changing the design (S205).

The management system 5 then notifies the edge computer 40 of the equipment system 2 of the content of control (S206). As a result, the edge computer 40 executes a change in the logic of the PLC 30 and the like and the PLC 30 executes a process according to a new logic and the like, whereby a change of the water treatment process and the like are executed. Note that the management system 5 can also output the content of control generated by simulation and the like to the management apparatus, a display, and the like.

### Effects

As described above, the water treatment system 1 according to the first embodiment enables flexible expansion of a module in response to a device expansion request in accordance with a demand. The water treatment system 1 enables data collected from individual water treatment devices used in various water treatment facilities to be diverted mutually without depending on the configuration of a water treatment device and thus the water treatment system 1 enables optimal control.

The water treatment system 1 is able to instantaneously calculate a module or a combination to operate without depending on experiences and the like and automatically propose the module or a combination by using the edge computer 40 and performing management at a module level. The water treatment system 1 is able to estimate an input/output value without testing actual equipment by simulating a method for addressing a sudden environmental variation on a virtual system in equipment with a little environmental variation.

The sampling apparatus 70 includes the first pipe 71 into which water flows, the valve 718 between the analysis apparatus 74 that performs analysis on the water that flows into and the first pipe 71, the second pipe 72 into which water flows, and the valve 728 between the analysis apparatus 74 and the second pipe 72. This enables the single sampling apparatus 70 to analyze both of the supplied water and the processed water, which makes it possible to efficiently acquire information on quality of water in the water treatment system.

### Second Embodiment

In the first embodiment, an example in which one edge computer 40 controls the entire water treatment device group 20 via the PLC 30 in the equipment system 2 has been described; however, the control is not limited to this. For example, an edge computer can be installed for each water treatment device in the water treatment device group 20 and the management system 5 can control the individual water treatment devices. Thus, in a second embodiment, an example in which the management system 5 directly controls each water treatment device and a configured module in the actual equipment will be described.

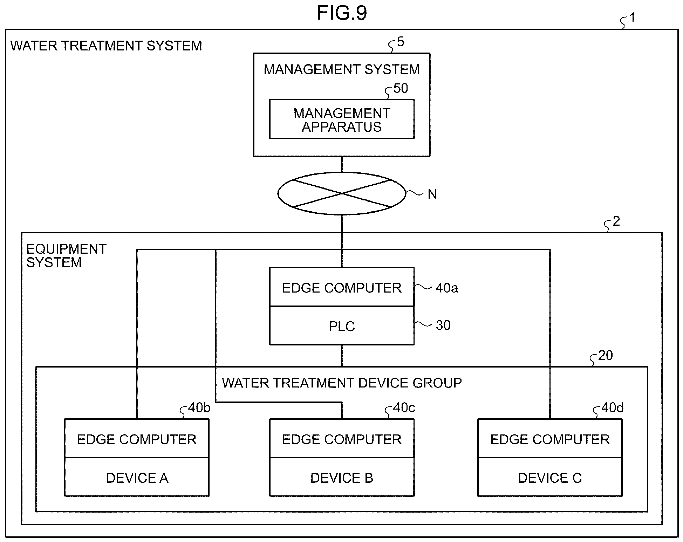

FIG. 9 is a diagram illustrating an architecture of the water treatment system 1 according to the second embodiment. As illustrated in FIG. 9, the water treatment system 1 according to the second embodiment includes the equipment system 2 and the management system 5 as in the first embodiment.

The difference from the first embodiment is that edge computers 40a, 40b, 40c, and 40d are provided for the PLC 30, and each of a device A, a device B, and a device C of the water treatment device group 20 in the equipment system 2. Note that the edge computers 40a, 40b, 40c, and 40d have a function similar to that of the edge computer 40 described in the first embodiment.

For example, the management system 5 acquires data on the state of the PLC 30 or each water treatment device, the control status, the control result, and the like from each of the edge computers 40a, 40b, 40c, and 40d. Then, the management system 5 generates content of control related to the operation of each water treatment device by using a virtual system, AI (machine learning model), and the like. The management system 5 then notifies each of the edge computers 40a, 40b, 40c, and 40d of the generated content of control.

As a result, each of the edge computers 40a, 40b, 40c, and 40d executes control in accordance with the control content of which each of the edge computers has been notified by the management system 5 for the PLC and each device. As described above, the management system 5 (AI engine 50b) directly controls the minimum unit of water treatment device in units of modules, thereby enabling more optimal control at high speed.

Furthermore, the management system 5 can obtain a solution that optimizes the entire processing system and thus optimize an operation in units of water treatment devices using each edge computer constituting a module, thereby combining modules. Furthermore, the management system 5 can directly send an instruction to the water treatment device and thereby the speed of operation to the water treatment device increases. Furthermore, it is possible to increase the degree of freedom of combination of water treatment devices, increase the degree of freedom of combination of parameters, and consider a causal relation between terminal devices. The management system 5 is able to quickly and appropriately derive a countermeasure against an unexpected variation.

### Third Embodiment

In the first embodiment and the second embodiment, the example where the single management system 5 manages and controls the single water treatment system (equipment system 2) has been described; however, the control is not limited to this. For example, the single management system 5 manages an integrated system obtained by combining a plurality of water treatment devices as a single water treatment device and thereby enables perspective control on the entire water treatment system.

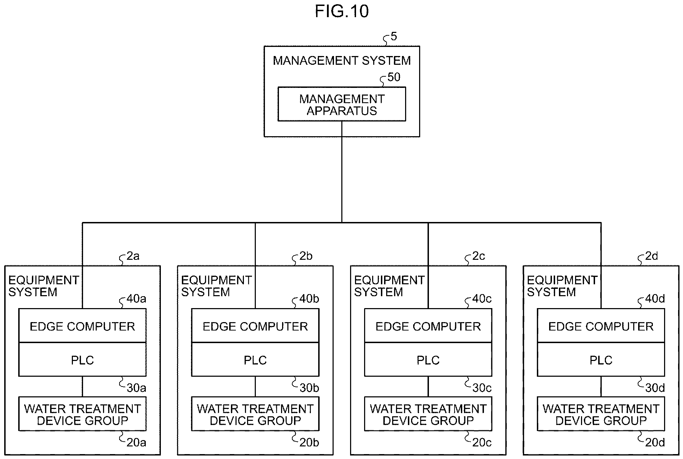

FIG. 10 is a diagram illustrating an architecture of the water treatment system 1 according to a third embodiment. As illustrated in FIG. 10, the water treatment system 1 according to the third embodiment includes the management system 5 and a plurality of equipment systems 2a, 2b, 2c, and 2d.

The management system 5 has a function similar to that of the management system 5 described in the first embodiment and each of the equipment systems 2a, 2b, 2c, and 2d has a function similar to that of the equipment system 2 described in the first embodiment.

The equipment system 2a includes a water treatment device group 20a, a PLC 30a, and an edge computer 40a and the equipment system 2b includes a water treatment device group 20b, a PLC 30b, and an edge computer 40b. The equipment system 2c includes a water treatment device group 20c, a PLC 30c, and an edge computer 40c and the equipment system 2d includes a water treatment device group 20d, a PLC 30d, and an edge computer 40d.

Note that, the water treatment device groups 20a, 20b, 20c, and 20d have a configuration similar to that of the water treatment device group 20 described in the first embodiment. The PLCs 30a, 30b, 30c, and 30d have a function similar to that of the PLC 30 described in the first embodiment. The edge computers 40a, 40b, 40c, and 40d have a function similar to that of the edge computer 40 described in the first embodiment.

In such a configuration, the management system 5 collects data not only from a single water treatment system but from a plurality of water treatment systems. For example, the management system 5 acquires various types of data on the water treatment process from each of the equipment systems 2a, 2b, 2c, and 2d. Then, the management system 5 generates content of control related to an integrated operation index of a plurality of equipment systems based on the operation status and the like of the water treatment process of each of the equipment systems. Thereafter, the management system 5 executes operation control on the water treatment process executed by each edge computer of each equipment system in accordance with the content of control.

For example, a water treatment system in which each equipment system 2 is installed in the same area is assumed. In this state, even when an instruction to increase a volume of production from the entire system is given, the management system 5 is able to make a change to content of control for increasing the operation rate of the equipment system 2b having a volume of production to spare. As a result, the management system 5 makes it possible to increase the volume of production by distributing a load on the entire water treatment system and thereby avoid a risk associated with stop of any water treatment system.

In the above-described state, even when an instruction to increase the volume of production from the equipment system 2a is given, the management system 5 is able to make a change to content of control for increasing the operation rate of the equipment system 2c having the lowest operation costs. As a result, the management system 5 enables an increase in the volume of production while executing cost reduction of the entire water treatment system.

The management system 5 is able to collect and integrally manage the operation status, capital investment, costs, and the like of each equipment system 2. As a result, when an operation load on a certain equipment system is abnormally high, the management system 5 can also propose a client for using another equipment system.

Furthermore, the management system 5 manufactures the equipment system 2a and the equipment system 2b that meet a request from the client and executes the water treatment process. Thereafter, even when the need to generate a new equipment system arises in association with a change in the request from the client, the management system 5 is able to respond to the request from the client while reducing the costs of generating the new system by proposing use of the equipment system 2d having the same configuration.

As described above, the water treatment system 1 according to the third embodiment enables data collected from a plurality of water treatment systems to be mutually diverted and optimized not in association with individual water treatment systems (equipment systems).

### Fourth Embodiment

The embodiments of the present disclosure have been described; however, the disclosure may be implemented in various different modes other than the above-described embodiments.

### Numerical Value and the Like

The number of water treatment devices, the number of equipment systems, specific examples of content of control, and the like described in the above-described embodiments are examples only and they can be optionally changed. Furthermore, in the flowcharts described in the embodiment, the order of processing is changeable within a range without contradiction.

### System

Information including the process procedures, the control procedures, the specific names, and various types of data and parameters in the document above and the drawings is optionally changeable unless otherwise specified. For example, the water treatment system 1 may be configured such that actual equipment automatically simulates an optimized virtual system configuration calculated by the management system 5 (plant-level-system and software).

Furthermore, each component of each illustrated apparatus is functionally conceptual, and is not necessarily required to be physically configured as illustrated in the drawings. That is, a specific mode of distribution and integration of each apparatus is not limited to that illustrated in the drawings. That is, all or a part thereof can be functionally or physically distributed and integrated in any unit in accordance with various types of loads, the status of use, and the like.

Moreover, all or any part of each processing function performed in each apparatus can be implemented by a central processing unit (CPU) and a program analyzed and executed by the CPU or can be implemented as hardware using a wired logic.

### Hardware

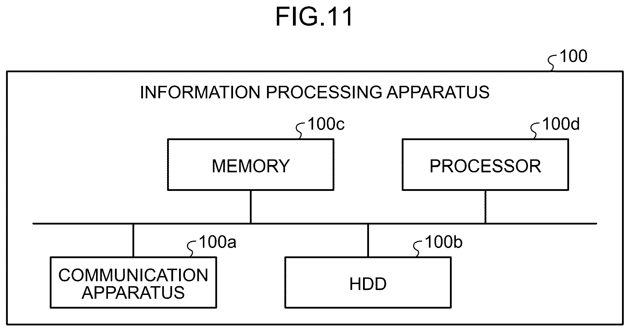

Next, an example of hardware configurations of the computers described in the embodiments will be described. Note that, since the management apparatus 50 and the edge computer 40 have similar hardware configurations, the management apparatus 50 and the edge computer 40 will be described as an information processing apparatus 100. FIG. 11 is a diagram illustrating an example of the hardware configuration. As illustrated in FIG. 11, the information processing apparatus 100 includes a communication apparatus 100a, a hard disk drive (HDD) 100b, a memory 100c, and a processor 100d. Furthermore, the units illustrated in FIG. 11 are connected to each other by a bus or the like.

The communication apparatus 100a is a network interface card or the like and communicates with another server. The HDD 100b stores a program for causing the functions illustrated in FIG. 3 to operate and a DB.

The processor 100d reads a program for executing processing similar to that of the processing units illustrated in FIG. 3 from the HDD 100b or the like and loads the program in the memory 100c, thereby operating a process for executing each function described with reference to FIG. 3 and the like. For example, when described by taking the management apparatus 50 as an example, the process executes a function similar to that of each processing unit of the management apparatus 50. Specifically, the processor 100d reads a program having functions similar to those of the virtual processing unit 53a, the control management unit 53b, and the like from the HDD 100b and the like. Then, the processor 100d executes a process of executing processing similar to that of the virtual processing unit 53a, the control management unit 53b, and the like.

As described above, the information processing apparatus 100 operates as an information processing apparatus that executes a measurement method by reading and executing the program. The information processing apparatus 100 can also implement a function similar to those of the above-described embodiments by reading the above-described program from a recording medium with a medium reading apparatus and executing the read program. Note that the program in other embodiments is not limited to being executed by the information processing apparatus 100. For example, the present disclosure can be similarly applied even to the case where another computer or another server executes a program or the case where the computer and the server execute the program cooperatively.

The program can be distributed via a network such as the Internet. Furthermore, the program is recorded in a computer-readable recording medium such as a hard disk, a flexible disk (FD), a CD-ROM, a magneto-optical (MO) disk, or a digital versatile disc (DVD), and the program can be executed by being read from the recording medium by the computer.

Some examples of a combination of the disclosed technical features will be described below.

According to the disclosure, it is possible to acquire information on quality of water efficiently in the water treatment system.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. A sampling apparatus (70) comprising:
a first pipe (71) into which water flows;
a first valve (718) between an analysis apparatus that performs analysis on the water that flows into and the first pipe (71);
a second pipe (72) into which water flows; and
a second valve (728) between the analysis apparatus and the second pipe (72).

2. The sampling apparatus (70) according to claim 1, wherein the first pipe (71) receives inflow of supplied water in a water treatment process and the second pipe (72) receives inflow of treated water in the water treatment process.

3. The sampling apparatus (70) according to claim 1 or 2, further comprising a cleaner that cleans both of the first pipe (71) and the second pipe (72) with both of the fist valve and the second valve (728) being closed.

4. The sampling apparatus (70) according to claim 3, wherein the cleaner cleans the first pipe (71) and the second pipe (72) by ultrasound.

5. A control method performed by a computer to control a sampling apparatus (70) including
a first pipe (71) into which water flows;
a first valve (718) between an analysis apparatus that performs analysis on the water that flows into and the first pipe (71);
a second pipe (72) into which water flows; and
a second valve (728) between the analysis apparatus and the second pipe (72),
wherein the method comprises controlling opening or closing the first valve (718) and the second valve (728).

6. The control method according to claim 5, wherein the controlling includes performing control such that overflow water is kept caused from the first pipe (71) when the first valve (718) is opened.

7. The control method according to claim 5 or 6, wherein the controlling includes alternately performing control for closing the first valve (718) and opening the second valve (728) and control for opening the first valve (718) and closing the second valve (728) at regular intervals.

8. The control method according to any one of claims 5 to 7, wherein the controlling includes inputting a signal of an open-close status of the first valve (718) and the second valve (728) together with a result of analysis by the analysis apparatus to a machine learning model and further performing analysis.

9. A control program that causes a computer to control a sampling apparatus (70) including
a first pipe (71) into which water flows;
a first valve (718) between an analysis apparatus that performs analysis on the water that flows into and the first pipe (71);
a second pipe (72) into which water flows; and
a second valve (728) between the analysis apparatus and the second pipe (72),
wherein the computer is caused to control opening or closing the first valve (718) and the second valve (728).
